# EUROPÄISCHE PATENTSCHRIFT

(11) **EP 0 778 878 B1**
(45) Veröffentlichungstag und Bekanntmachung des Hinweises auf die Patenterteilung: **20.10.1999**
(21) Anmeldenummer: 95930521.0
(22) Anmeldetag: 24.08.1995
(51) Int. Cl.: C11D 1/72, C08G 65/32, C07C 43/00

(54) **VERWENDUNG VON METHYL-ENDGRUPPENVERSCHLOSSENEM ALKYL- UND/ODER ALKENYLPOLYGLYCOLETHER ZUR HERSTELLUNG VON OBERFLÄCHENAKTIVEN MITTELN**
USE OF METHYL END GROUP CAPPED ALKYL AND/OR ALKENYL POLYGLYCOL ETHERS FOR THE MANUFACTURE OF SURFACE ACTIVE AGENTS
UTILISATION DE POLYGLYCOLETHERS D'ALKYLE ET/OU D'ALCENYLE BLOQUES PAR UN GROUPE TERMINAL METHYLE POUR L'OBTENTION D' AGENTS TENSIO-ACTIFS

(30) Priorität: 01.09.1994 DE 4431158
(43) Veröffentlichungstag der Anmeldung: 18.06.1997
(73) Patentinhaber: Henkel Kommanditgesellschaft auf Aktien, 40191 Düsseldorf (DE)
(72) Erfinder: SCHMID, Karl, D-40822 Mettmann (DE); BIGORRA LLOSAS, Joaquim, E-08203 Sabadell (ES)
(86) Internationale Anmeldenummer: EP9503359
(87) Internationale Veröffentlichungsnummer: WO9606905

(56) Entgegenhaltungen:
- EP-A- 0 012 052
- EP-A- 0 161 537
- EP-A- 0 180 081
- EP-A- 0 202 638
- EP-A- 0 590 722
- DE-A- 2 243 307
- DE-C- 3 744 525

## Beschreibung

Die Erfindung betrifft die Verwendung von methyl-endgruppenverschlossenen Alkyl und/oder Alkenylpolyglycolethern zur Herstellung von oberflächenaktiven Mitteln.

### Stand der Technik

Für eine Vielzahl technischer Prozesse ist die Anwesenheit von Schaum äußerst unerwünscht. So kommt es z. B. sowohl bei der maschinellen Reinigung von Bier- oder Milchflaschen als auch der Spritzreinigung von Automobilblechen nicht nur auf die reinigende bzw. entfettende Wirkung der eingesetzten oberflächenaktiven Mittel an, die Vermeidung von Schaum, der die Anlagenfunktionen stark beeinträchtigen kann, ist von gleich großer Bedeutung. Dies gilt um so mehr, als in vielen Fällen hochaktive, jedoch auch starkschäumende anionische Tenside eingesetzt werden.

Das Problem der Schaumregulierung ist freilich seit langer Zeit bekannt und dementsprechend sind aus dem Stand der Technik eine Vielzahl von mehr oder weniger überzeugenden Problemlösungen bekannt, die in zwei Gruppen eingeteilt werden können:

Bei der einen handelt es sich um Verfahren unter Zugabe von Entschäumern, bei denen es sich vielfach um paraffinische Kohlenwasserstoffe oder Siliconverbindungen handelt. Für die beschriebenen Anwendungen ist dies jedoch in den meisten Fällen unerwünscht. Bei der zweiten Gruppe von Verfahren handelt es sich um den Einsatz von oberflächenaktiven Mitteln, die selbst schaumarm sind und gegebenenfalls zusätzlich noch über entschäumende Eigenschaften verfügen. In der Regel handelt es sich hierbei um nichtionische Tenside oder tensidähnliche Systeme, wie beispielsweise Fettalkoholpropylenglycolether oder Blockpolymere von Ethylen- und Propylenglycol, die allerdings keine ausreichende biologische Abbaubarkeit aufweisen.

Als besonders effektive schaumarme Tenside haben sich endgruppenverschlossene Fettalkoholpolyglycolether, sogenannte "Mischether" am Markt etabliert, die beispielsweise von R. Piorr in **Fat.Sci.Technol. 89, 106 (1987)** beschrieben werden. Bei diesen Produkten handelt es sich in der Regel um butylendgruppenverschlossene Niotenside, wie sie beispielsweise aus den Schriften **EP-A 0124815, EP-B 0303928, EP-B 0324340, EP-A 0420802, DE-A 3928600** und **DE-C 4243643** bekannt sind.

Eine Sonderstellung nehmen Methylmischether ein, die einen Methyl-Endgruppenverschluß aufweisen und üblicherweise durch Umsetzung der entsprechenden Fettalkoholpolyglycolether mit Methylhalogeniden [**US 4587365, BASF**] oder Dimethylsulfat [**EP-B 0302487, BASF**] hergestellt werden.

So ist beispielsweise aus der **EP-A 0161537** (BASF) die Verwendung u.a. von methyl-endgruppenverschlossenen Mischethern für die maschinelle Flaschenreinigung bekannt. In der **EP-B 0180081** (BASF) werden die gleichen Stoffe als Entschäumer für die Zucker- und Hefeindustrie vorgeschlagen. Gegenstand der **EP-B 0202638** (BASF) sind schließlich flüssige Tensidkonzentrate für stark alkalische Reinigungsmittel, enthaltend Methylmischether, Alkylpolyglucoside, Anlagerungsprodukte von Maleinsäureanhydrid an ungesättigte Fettsäuren sowie verzweigte Fettsäuren.

Die bislang bekannten Methylmischether haben bezüglich des Schaumvermögens und insbesondere Entschäumungsverhalten gegenüber den weit verbreiteten Butylmischethern stets deutlich schlechter abgeschnitten. Infolge der limitierten Verfügbarkeit von Butylchlorid als geeignetem Reagenz für den C₄-Endgruppenverschluß von Alkylpolyglycolethern hat das Marktbedürfnis nach Methylmischethern mit verbesserten anwendungstechnischen Eigenschaften, die einfach und preiswert herzustellen sind, in den letzten Jahren jedoch stark zugenommen.

Die Aufgabe der Erfindung hat somit darin bestanden, neue methyl-endgruppenverschlossene Alkylpolyglycolether zur Verfügung zu stellen, die frei von den geschilderten Nachteilen sind und hinsichtlich Schaum, Entschäumung, Reinigungsvermögen und biologischer Abbaubarkeit butyl-endgruppenverschlossenen Handelsprodukten zumindest ebenbürtig sind.

### Beschreibung der Erfindung

Gegenstand der Erfindung ist die Verwendung von methyl-endgruppenverschlossenen Alkyl- und/oder Alkenylpolyglycolethern der Formel **(I),** 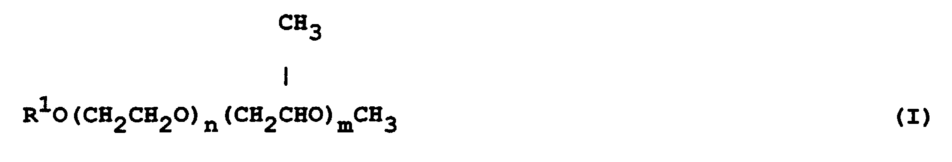 in der R¹ für einen linearen oder verzweigten Alkyl- und/oder Alkenylrest mit 6 bis 22 Kohlenstoffatomen, n für Zahlen von 4 bis 15 und m für Zahlen von 0,5 bis 1,5 steht, zur Herstellung von schaumarmen Reinigungsmitteln sowie Entschäumern für die Zucker- und Hefeindustrie.

Überraschenderweise wurde gefunden, daß Methylmischether, die eine Abfolge von durchschnittlich 4 bis 15 Mol Ethylenoxid und 0,5 bis 1,5 Mol Propylenoxid in der Polyalkylenoxidkette aufweisen, gegenüber bekannten Methyl- und z.T. auch Butylmischethern verbesserte schaumdrückende Eigenschaften aufweisen. Sie zeichnen sich ferner durch ein sehr geringes eigenes Schaumvermögen, gute Reinigungsleistung und zufriedenstellende biologische Abbaubarkeit aus.

Zur Herstellung von methyl-endgruppenverschlossenen Alkyl-und/oder Alkenylpolyglycolethern der Formel **(I)** 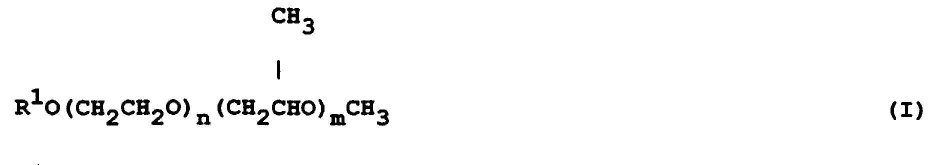 in der R¹ für einen linearen oder verzweigten Alkyl- und/oder Alkenylrest mit 6 bis 22 Kohlenstoffatomen, n für Zahlen von 4 bis 15 und m für Zahlen von 0,5 bis 1,5 steht, werden an primäre Alkohole der Formel **(II)**

**R**^{**1**}**OH** **(II)**

in an sich bekannter Weise zunächst durchschnittlich n Mol Ethylenoxid und anschließend m Mol Propylenoxid anlagert - wobei R¹, n und m die oben genannten Bedeutungen aufweisen - und die resultierenden Polyglycolether anschließend mit einem Methylierungsmittel umgesetzt.

### Primäre Alkohole

Als primäre Alkohole können beispielsweise **Fettalkohole** der Formel **(II)** eingesetzt, in der R¹ für einen Alkyl- und/oder Alkenylrest mit 8 bis 18 Kohlenstoffatomen steht.

Typische Beispiele sind Capronalkohol, Caprylalkohol, 2-- Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern- oder Talgfettalkohol.

Als primäre Alkohole kommen ferner **Guerbetalkohole** der Formel **(II)** in Frage, in der R¹ für einen in 2-Stellung verzweigten Alkylrest mit 16 bis 20 Kohlenstoffatomen steht. Guerbetalkohole werden üblicherweise durch basische Kondensation von Fettalkoholen hergestellt. Eine Übersicht zu diesem Thema ist von O'Lenick und Bibo in **Soap, Cosm.Chem.Spec. April 52 (1987)** erschienen. Typische Beispiele sind 2-Hexyldodecanol und 2-Octyltetradecanol sowie deren Mischungen auf Basis von technischen C₈-C₁₀-Fettalkoholen. Die letztgenannten Produkte zeichnen sich durch ein besonders hohes Reinigungsvermögen und sehr gute biologische Abbaubarkeit aus.

### Ethoxylierung und Propoxylierung

Die Ethoxylierung und Propoxylierung der genannten Alkohole kann in an sich bekannter Weise bei Temperaturen von 120 bis 180°C in Anwesenheit basischer Katalysatoren wie beispielsweise Natriummethylat oder calciniertem Hydrotalcit durchgeführt werden. Demzufolge können die Alkoxylate sowohl eine konventionelle als auch eine eingeengte Homologenverteilung aufweisen. Es sei noch einmal ausdrücklich darauf hingewiesen, daß die Abfolge von Ethoxylierung und Propoxylierung kritisch für die anwendungstechnischen Eigenschaften der Produkte ist. Methyl-endgruppenverschlossene Mischether, die im Sinne der Erfindung Verwendung finden sollen, werden ausschließlich auf Basis von Alkyl- und/oder Alkenylpolyglycolethern erhalten, die zunächst einen Block von Ethylenoxideinheiten und dann einen geringen Anteil von Propylenoxid-Einheiten aufweisen. Besonders bevorzugt sind hierbei Mischether auf Basis von Alkyl- und/oder Alkenylpolyglycolethern mit durchschnittlich 4 bis 12 Mol Ethylenoxid und 0,5 bis 1,5 Mol Propylenoxid.

### Methyl-Endgruppenverschluß

Auch der Methyl-Endgruppenverschluß kann in an sich bekannter Weise, d.h. unter Einsatz von Methylchlorid bzw. Dimethylsulfat analog der Schriften US 4587365 bzw. EP-B 0302487 erfolgen. Zur Durchführung der Reaktion empfiehlt es sich, eine Temperatur im Bereich von 60 bis 120 und vorzugsweise 80 bis 100°C einzuhalten. Der Endgruppenverschluß stellt eine Williamson'sche Ethersynthese dar, die nur in Gegenwart mindestens stöchiometrischer Mengen einer starken Base, wie beispielsweise Natriumhydroxid oder insbesondere Kaliumhydroxid gelingt. Es hat sich weiterhin als vorteilhaft erwiesen, Alkyl- und/oder Alkenylpolyglycolether, Base und Methylierungsmittel im molaren Verhältnis von 1 : (1,5 - 2,0) : (1,5 - 2,0) einzusetzen.

### Gewerbliche Anwendbarkeit

Die Methylmischether sind besonders schaumarm und zeichnen sich durch sehr gute entschäumende Eigenschaften, hohe Reinigungsleistung sowie gute ökotoxikologische Verträglichkeit aus.

### Oberflächenaktive Mittel

Die Methylmischether werden im Sinne der Erfindung verwendet zur Herstellung von :
*** Mittel für die maschinelle Flaschenreinigung, insbesondere für die Reinigung von Bier- und Milchflaschen.
*** Mittel für die Reinigung harter Oberflächen, insbesondere für das "Cleaning-in-place (CIP)".
*** Mittel für die Entschäumung von Zwischenprodukten bei der Zucker- und Hefeherstellung.

Unter Reinigungsmitteln werden im Zusammenhang mit der Erfindung einmal die zur direkten Anwendung auf die zu reinigenden Substrate bestimmten wäßrigen Lösungen verstanden, daneben umfaßt der Begriff aber auch die zur Herstellung der Anwendungslösungen bestimmten Konzentrate und gegebenenfalls festen Mischungen. Die gebrauchsfertigen Lösungen können sauer bis stark alkalisch eingestellt sein und werden in der Regel bei Temperaturen von etwa 20 bis 90°C eingesetzt.

### Hilfs- und Zusatzstoffe

Die schaumarmen Reinigungsmittel können weitere Hilfs- und Zusatzstoffe und insbesondere weitere anionische, nichtionische, kationische und/oder amphotere bzw. zwitterionische Tenside enthalten.

Typische Beispiele für **anionische Tenside** sind Alkylbenzolsulfonate, Alkansulfonate, Olefinsulfonate, Alkylethersulfonate, Glycerinethersulfonate, ∝-Methylestersulfonate, Sulfofettsäuren, Alkylsulfate, Fettalkoholethersulfate, Glycerinethersulfate, Hydroxymischethersulfate, Monoglycerid(ether) sulfate, Fettsäureamid(ether) sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Alkyloligoglucosidsulfate, Proteinfettsäurekondensate (insbesondere pflanzliche Produkte auf Sojabasis) und Alkyl(ether)phosphate. Sofern die anionischen Tenside Polyglycoletherketten enthalten, können sie eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Butylmischether, Alk(en)yloligoglykoside, Fettsäure-N-alkylglucamide, Proteinhydrolysate (insbesondere pflanzliche Produkte auf Sojabasis) Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124 oder J.Falbe (ed.), "Katalysatoren, Tenside und Mineralöladditive", Thieme Verlag, Stuttgart, 1978, S.123-217** verwiesen.

An Buildern und Komplexbildnern kommen können die Mittel vor allem Alkalimetallorthophosphate, -polymerphosphate, -silicate, -borate, -carbonate, -polyacrylate und -gluconate, sowie Zeolithe, Schichtsilicate, Citronensäure, Nitrilotriessigsäure, Ethylendiamintetraessigsäure, 1-Hydroxyalkan-1,1-diphosphonsäuren, Ethylendiamintetra-(methylenphosphonsäure) sowie Phosphonoalkanpolycarbonsäuren wie z.B. Phosphonobutantricarbonsäure bzw. Alkalimetallsalze dieser Säuren enthalten.

Hochalkalische Reinigungsmittel, insbesondere für die Flaschenreinigung, können fener beträchtliche Mengen Ätzalkali in Form von Natrium - und/oder Kaliumhydroxid aufweisen. Wenn besondere Reinigungseffekte gewünscht werden, können die Mittel zudem organische Lösungsmittel, beispielsweise Alkohole, Benzinfraktionen sowie freie Alkanolamine enthalten.

Gemäß der Erfindung können die methyl-endgruppenverschlossenen Alkyl- und/ oder Alkenylpolyglycolether der Formel **(I)** zur Herstellung von schaumarmen Reinigungsmitteln sowie Entschäumern für die Zucker- und Hefeindustrie, in Mengen von 1 bis 50, vorzugsweise 5 bis 25 Gew.-% - bezogen auf die Mittel - verwendet werden.

Die folgenden Beispiele sollen den Gegenstand der Erfindung näher erläutern, ohne ihn darauf einzuschränken.

### Beispiele

### I. Allgemeine Herstellvorschrift für Mischether

In einen Dreihalskolben mit KPG-Rührer, Innenthermometer und Rückflußkühler werden jeweils 5,5 Mol des entsprechenden Alkyl-EO/PO-Adduktes und 770 g (11,25 mol) 82 Gew.-%iges, schuppenförmiges Kaliumhydroxid vorgelegt. Der Kolben wird zweimal evakuiert und mit Stickstoff gespült. Nach Zugabe von 11,25 Mol Methylchlorid bzw. Butylchlorid wird das Gemisch unter Rühren auf 80°C erwärmt und 8 h bei dieser Temperatur belassen. Danach wird überschüssiges Alkylierungsmittel bei einem Druck von 1 bar und einer maximalen Sumpftemperatur von 200°C abdestilliert, wobei Stickstoff als Treibgas eingesetzt wird.

Das Reaktionsgemisch wird danach auf 60°C abgekühlt und mit 2N-Schwefelsäure neutralisiert. Anschließend wird das Rohprodukt mit einer seinem Volumen entsprechenden Menge Wasser versetzt und bei 50°C belassen bis Phasentrennung eintritt. Durch Abtrennen der wäßrigen Phase wird das Reinprodukt erhalten.

Die nach dieser Vorschrift hergestellten Mischether M1 bis M3 sind erfindungsgemäß, die Mischether M4 bis M8 dienen dem Vergleich. Die Einzelheiten können Tabelle 1 entnommen werden.

**Tabelle 1**

| Methyl- und Butylmischether | | | | | |
|---|---|---|---|---|---|
| **Bsp.** | **R**^{**1**} | **Mol EO** | **Mol PO** | **Mol EO** | **EGV** |
| M1 | Octyl | 4 | 1 | - | Methyl |
| M2 | Dodecyl | 7 | 1,2 | - | Methyl |
| M3 | 2-Hexyldodecyl | 10 | 1,2 | 1 - | Methyl |
| M4 | Octyl | - | 1 | 4 | Methyl |
| M5 | Octyl | 2 | 1 | 2 | Methyl |
| M6 | Dodecyl | - | 1,2 | 7 | Methyl |
| M7 | Dodecyl | 7 | - | - | Butyl |
| M8 | Dodecyl | 3 | 1,2 | 4 | Butyl |
| Legende: EGV = Endgruppenverschluß | | | | | |

### II. Bestimmung der Entschäumerwirkung

**Methode.** In einem doppelwandigen 2-l-Meßzylinder werden 300 ml einer 1 Gew.-%igen Natriumhydroxidlösung auf 20 bzw. 65°C temperiert. Anschließend wird der jeweils ausgewählte schaumdrückende Zusatzstoff in den nachfolgend angegebenen Mengen zugesetzt. Mit Hilfe einer Laborschlauchpumpe wird die Flüssigkeit mit einer Umwälzgeschwindigkeit von 4 l/min umgepumpt. Dabei wird die Prüflösung ca. 5 mm über dem Boden des Meßzylinders mit Hilfe eines Glasrohres, das mit einer Pumpe verbunden ist, angesaugt und über ein zweites Glasrohr, das an der 2000-ml-Marke angebracht ist, in freiem Fall zurückgeführt.

Nach 30 s dosiert man zunächst 1 ml einer 1 Gew.-%igen wäßrigen Lösung von Tetrapropylenbenzolsulfonat-Triethanolammonium-Salz (nachfolgend als "Testschäumer TS" bezeichnet) in die Flotte und bestimmt nach weiteren 30 s das entstandene Volumen, das durch Flüssigkeit und Schaum gebildet wird. In Zeitabständen von jeweils 1 min wird nachfolgend weiterer Testschäumer zudosiert und das nach 30 s entstandene Schaum/ Flüssigkeits-Volumen bestimmt. Diesen stufenförmigen Zyklus von Zudosierung des Testschäumers und Schaummessung setzt man solange fort, bis die Tensidlösung im Meßzylinder auf 2000 ml aufgeschäumt ist.

Die Ergebnisse sind in den Tabellen 2 und 3 zusammengefaßt.

**Tabelle 2**

| Entschäumerwirkung (20°C) | | | | | | | | |
|---|---|---|---|---|---|---|---|---|
| **TS** **ml** | **M1** **ml** | **M2** **ml** | **M3** **ml** | **M4** **ml** | **M5** **ml** | **M6** **ml** | **M7** **ml** | **M8** **ml** |
| 0 | 300 | 300 | 300 | 300 | 300 | 300 | 300 | 300 |
| 1 | 300 | 300 | 300 | 300 | 300 | 300 | 300 | 300 |
| 2 | 300 | 300 | 300 | 300 | 300 | 300 | 300 | 300 |
| 3 | 300 | 300 | 300 | 320 | 320 | 300 | 300 | 300 |
| 4 | 340 | 320 | 300 | 340 | 330 | 320 | 320 | 320 |
| 5 | 380 | 360 | 300 | 390 | 380 | 360 | 360 | 360 |
| 6 | 400 | 390 | 300 | 440 | 440 | 390 | 390 | 390 |
| 7 | 420 | 410 | 420 | 470 | 480 | 430 | 430 | 430 |
| 8 | 420 | 410 | 420 | 490 | 490 | 450 | 450 | 450 |
| 9 | 460 | 430 | 430 | 530 | 520 | 470 | 470 | 470 |
| 10 | 480 | 460 | 470 | 570 | 550 | 490 | 480 | 490 |
| 11 | 500 | 490 | 490 | 590 | 590 | 520 | 520 | 510 |
| 12 | 540 | 520 | 510 | 630 | 630 | 560 | 550 | 560 |
| 13 | 580 | 550 | 550 | 690 | 680 | 590 | 590 | 590 |
| 14 | 600 | 580 | 570 | 780 | 750 | 640 | 630 | 620 |
| 15 | 660 | 650 | 660 | 950 | 940 | 700 | 720 | 700 |
| 16 | 720 | 700 | 710 | 1150 | 1100 | 890 | 820 | 880 |
| 17 | 800 | 790 | 810 | 1700 | 1650 | 990 | 990 | 980 |
| 18 | 1000 | 950 | 990 | 2000 | 2000 | 1300 | 1290 | 1400 |
| 19 | 1300 | 1250 | 1270 | | | 2000 | 1650 | 2000 |
| 20 | 1680 | 1500 | 1530 | | | | 2000 | |
| 21 | 2000 | 2000 | 2000 | | | | | |

**Tabelle 3**

| Entschäumerwirkung (65°C) | |
|---|---|
| **M** | **ml Testschäumer bis Volumen 2000 ml** |
| M1 | 20 |
| M2 | 20 |
| M3 | 20 |
| M4 | 15 |
| M5 | 14 |
| M6 | 15 |
| M7 | 18 |
| M8 | 15 |

## Patentansprüche

1. Verwendung von methyl-endgruppenverschlossenen Alkylund/oder Alkenylpolyglycolethern der Formel (I), 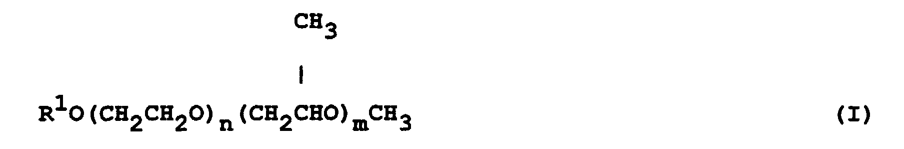 in der R¹ für einen linearen oder verzweigten Alkylund/oder Alkenylrest mit 6 bis 22 Kohlenstoffatomen, n für Zahlen von 4 bis 15 und m für Zahlen von 0,5 bis 1,5 steht, zur Herstellung von schaumarmen Reinigungsmitteln sowie Entschäumern für die Zucker- und Hefeindustrie.

## Claims

1. The use of methyl-end-capped alkyl and/or alkenyl polyglycol ethers corresponding to formula (I): 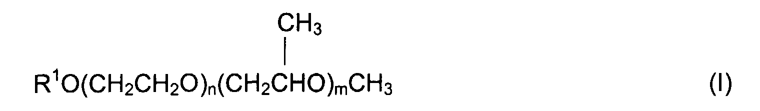 in which R¹ is a linear or branched alkyl and/or alkenyl group containing 6 to 22 carbon atoms, n is a number of 4 to 15 and m is a number of 0.5 to 1.5,
for the production of low-foaming cleaning formulations and defoamers for the sugar and yeast industry.

## Revendications

1. Utilisation de polyglycoléthers d'alkyle et/ou d'alcényle comportant un groupe terminal de fermeture méthyle de la formule **(I),** 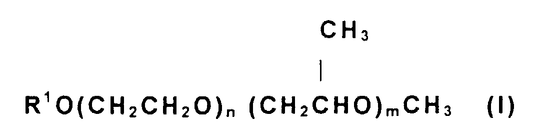 dans laquelle R¹ représente un radical alkyle et/ou alcényle linéaire ou ramifié comportant 6 à 22 atomes de carbone, n correspond à un nombre de 4 à 15 et m, à un nombre de 0,5 à 1,5, pour la production de nettoyants à mousse freinée ainsi que d'agents antimousse pour l'industrie du sucre et de la levure.
